(19) Europäisches Patentamt
European Patent Office
Office européen des brevets

(11) Publication number: 0 428 772 A1

(12) **EUROPEAN PATENT APPLICATION**

(21) Application number: 89121515.4

(22) Date of filing: 21.11.89

(51) Int. Cl.5: A61K 31/55, //(A61K31/55, 31:495)

(43) Date of publication of application: 29.05.91 Bulletin 91/22

(84) Designated Contracting States: AT BE CH LI LU NL SE

(71) Applicant: Thornfeldt, Carl Richard
1054 N.W. 2nd Avenue
Ontario Oregon 97914(US)

Applicant: Thornfeldt, Robert Ernest
1021 S.W. 5th Avenue
Ontario Oregon 97914(US)

(72) Inventor: Thornfeldt, Carl Richard
1054 N.W. 2nd Avenue
Ontario Oregon 97914(US)
Inventor: Thornfeldt, Robert Ernest
1021 S.W. 5th Avenue
Ontario Oregon 97914(US)

(74) Representative: Reinhard, Skuhra, Weise
Friedrichstrasse 31
W-8000 München 40(DE)

(54) **Treatment of colic and teething.**

(57) Teething and colic are alleviated in infants and toddlers by the administration of a combination of an antihistamine and a benzodiazepine having sedative hypnotic activity. An exemplary antihistamine is hydroxyzine pamoate, and an exemplary benzodiazepine is diazepam.

EP 0 428 772 A1

# TREATMENT OF COLIC AND TEETHING

## BACKGROUND OF THE INVENTION

This invention relates to the treatment of colic and teething, two conditions that afflict a significant portion of babies and young children.

Colic is a symptom complex characterized by paroxysms of presumably severe abdominal pain and crying with irritability and fussing in an otherwise healthy infant. These episodes usually occur during the first one and one-half to nine months of life and afflict up to 40% of infants. There are a number of known and presumed causes of this condition including milk allergy, under feeding, over feeding, high carbohydrate diet, ineffective burping, excessively large holes in the nipple, excess air sucked in, changes in handling, feeding, and sleeping routines, stimulus overload, anxious parents, and gastrointestinal smooth muscle cramping. To be effective, therapeutic drugs must do more than merely relieve pain, since pain plays a minor role if any in colic.

Teething is another symptom complex occurring in nearly all infants and toddlers to some degree at three to eighteen months of age. Teething is characterized by pain, tenderness, edema, excess salivation, irritability and insomnia. These symptoms result from migration and eventual eruption of deciduous teeth through the oral mucosa.

Current therapies for colic are generally discouraging, especially for severe episodes. On a short-term basis, mechanical maneuvers such as rhythmic motions are often beneficial. Pharmaceutical intervention, however, should be reserved for prolonged episodes, i.e., those exceeding three weeks. Attempts with the latter have included sedation with either antihistamines or phenobarbital, a long-acting barbiturate. Unfortunately, these have met with limited success. Phenobarbital is particularly undesirable since it usually produces excessive irritability when the sedation subsides.

Another seemingly likely candidate would be dicyclomine, a tertiary amine with antimuscarinic activity that has a published efficacy of 63%. Unfortunately, dicyclomine is limited by a narrow margin of safety due to its known induction of apnea, seizures, and coma. As a result, it is absolutely contraindicated in babies less than six months of age.

In the case of teething, known therapies include providing firm, blunt or cool objects for the baby to bite on, and very short-acting topical anesthetics. There are no other treatment regimens except sedation.

Two combinations of an antihistamine with a barbiturate have been used in the treatment of teething and colic and have demonstrated efficacy for both conditions.

The first is the combination of diphenhydramine (2-diphenylmethoxy-N,N-dimethylethanamine hydrochloride; available from Parke Davis & Co., Morris Plains, New Jersey, as Benadryl Elixir) and butabarbital sodium (5-ethyl-5-(1-methylpropyl)-2,4,6(1H,3H,5H)pyrimidinetrione sodium salt; available from Wallace Laboratories, Cranbury, New Jersey, in both powder form and as Butisol Elixir). The combination has been formulated by mixing 650mg butabarbital sodium powder in enough water to form a paste, then combining the paste with 120mL of Benadryl Elixir which contains 12.5mg diphenhydramine per 5mL including 14% ethanol, and finally adding 3 drops of hydrochloric acid to clear the solution. The dosage is $\frac{1}{2}$ -1 teaspoonful diluted in $\frac{1}{2}$ -ounce milk or juice administered orally at the onset of symptoms and repeated every 4-8 hours as needed.

The second combination is that of promethazine hydrochloride (N,N, α-trimethyl-10H-phenothiazine-10-ethanamine; available from Wyeth Laboratories, Philadelphia, Pennsylvania, as Phenergan syrup) and butabarbital sodium. This combination has been formulated by mixing equal parts of Butisol Elixir (which contains 30mg butabarbital sodium per 5mL including 7% ethanol) and Phenergan syrup (which contains 6.25mg promethazine hydrochloride per 5mL including 7% ethanol). The dosage is 1-2 teaspoonsful administered orally at the onset of symptoms and repeated every 4-8 hours as needed.

These two combinations have been administered by the inventors herein as therapy to patients in a pediatrician's office in Ontario, Oregon, as part of their practice in pediatrics over a period of fifteen years prior to the filing date of the present application.

## SUMMARY OF THE INVENTION

It has now been discovered that the combination of an antihistamine and a benzodiazepine having sedative hypnotic activity is effective as a therapeutic composition for teething, colic or both.

## DETAILED DESCRIPTION OF THE INVENTION

In specific terms, the antihistamines contemplated within the present invention are histamine 1 receptor antagonists. Those of greatest interest in the present invention are the class known as ataractics, i.e., species displaying activity against ataraxia. Preferred among this class are cyclizine derivatives, notably those having the formula

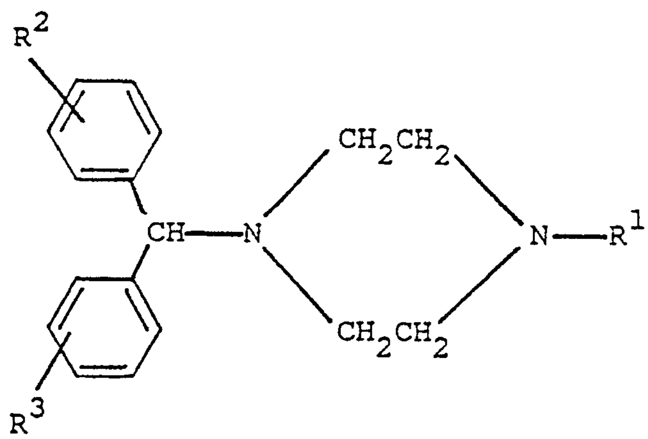

(I)

in which:

$R^1$ is $C_1$-$C_6$ alkyl, hydroxy-($C_1$-$C_6$ alkyl), hydroxy-($C_1$-$C_6$ alkoxy)-($C_1$-$C_6$ alkyl), phenyl-($C_1$-$C_6$ alkyl), or ($C_1$-$C_6$ alkyl)-phenyl-($C_1$-$C_6$ alkyl); and

$R^2$ and $R^3$, which may be the same or different, are a hydrogen atom, a halogen atom, or $C_1$-$C_6$ alkyl;

and acid salts thereof.

Within the scope of formula I, certain species are preferred. These are the species in which $R^1$ is $C_1$-$C_3$ alkyl, hydroxy-($C_1$-$C_3$ alkyl), hydroxy-($C_1$-$C_3$ alkoxy)-($C_1C_3$ alkyl), phenyl-($C_1$-$C_3$ alkyl) or ($C_1$-$C_4$ alkyl)-phenyl-($C_1$-$C_3$ alkyl); and $R^2$ and $R^3$ are a hydrogen atom, a chlorine atom, a bromine atom or $C_1$-$C_3$ alkyl.

Further preferred are those in which $R^1$ is methyl, hydroxyethoxyethyl or ($C_1$-$C_4$ alkyl)-phenylmethyl; $R^2$ is a hydrogen atom, a chlorine atom or a bromine atom; and $R^3$ is a hydrogen atom.

In this disclosure, the term "alkyl" is intended to encompass both straight-chain and branched-chain groups. The term "halogen" is intended to include fluorine, chlorine, bromine and iodine atoms only. The term "acid salts" is intended to encompass both mineral acids and organic acids. Preferred acid salts are hydrochlorides, lactates and pamoates.

Examples of cyclizine derivatives within the scope of formula I and useful in the present invention are as follows (generic name followed by Chemical Abstracts name and substituents for formula I):

buclizine dihydrochloride; 1-[(4-chlorophenyl)phenyl-methyl]-4-[[4-(1,1-dimethylethyl)phenyl)methyl] piperazine dihydrochloride;

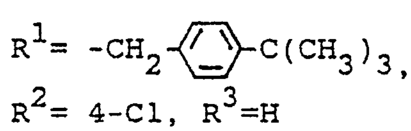

$R^2$= 4-Cl, $R^3$=H

chlorcyclizine hydrochloride; 1-[(4-chlorophenyl)phenylmethyl]-4-methylpiperazine hydrochloride; $R^1 = CH_3$, $R^2$ = 4-Cl, $R^3$ = H

cyclizine hydrochloride; 1-diphenylmethyl-4-methylpiperazine hydrochloride; $R^1 = CH_3$, $R^2$ = H, $R^3$ = H

cyclizine lactate; 1-diphenylmethyl-4-methylpiperazine lactate; $R^1 = CH_3$, $R^2$ = H, $R^3$ = H

hydroxyzine hydrochloride; 2-[2-[4-[(4-chlorophenyl)phenylmethyl]-1-piperazinyl]ethoxy]ethanol hydrochloride; $R^2 = -CH_2CH_2OCH_2CH_2OH$, $R^2$ = 4-Cl, $R^3$ = H

hydroxyzine pamoate; 2-[2-[4-[(4-chlorophenyl)phenylmethyl]-1-piperazinyl]ethoxy]ethanol pamoate; $R^1 = -CH_2CH_2OCH_2CH_2OH$, $R^2$ = 4-Cl, $R^3$ = H

meclizine hydrochloride; 1-[(4-chlorophenyl) phenylmethyl]-4-[(3-methylphenyl)methyl]piperazine hydrochlo-

ride;

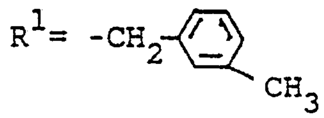

,

$R^2 = 4\text{-Cl}$, $R^3 = H$

Each of the examples in this list is a known, commercially available material.

Preferred among the benzodiazepines of the combination of the present invention are those having the formula

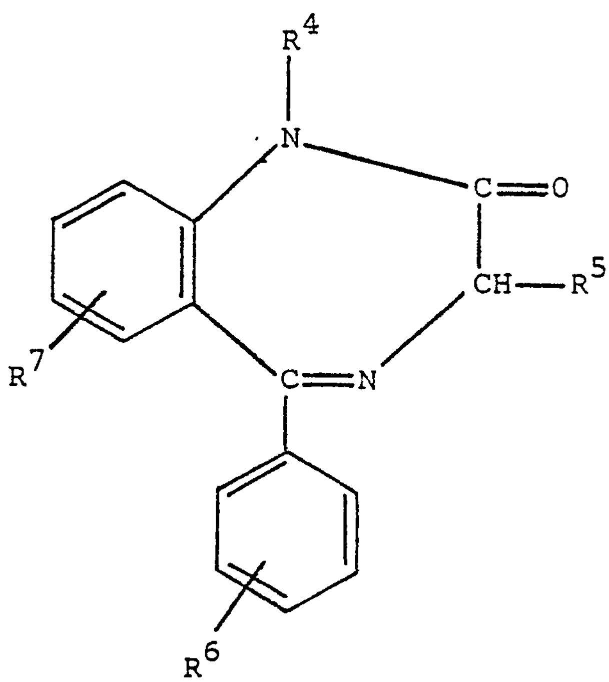

(II)

in which:

$R^4$ is a hydrogen atom, $C_1$-$C_6$ alkyl, $C_3$-$C_6$ cycloalkyl, $C_1$-$C_6$ haloalkyl, amino-($C_1$-$C_6$ alkyl), ($C_1$-$C_3$ alkylamino)-($C_1$-$C_6$ alkyl), or di($C_1$-$C_3$ alkylamino)-($C_1$-$C_6$ alkyl);

$R^5$ is a hydrogen atom, hydroxy or carboxy;

$R^6$ is a hydrogen atom, a halogen atom or $C_1$-$C_6$ alkyl; and

$R^7$ is a hydrogen atom, a halogen atom or $C_1$-$C_6$ alkyl;

and salts thereof.

Preferred species within the scope of formula II are those in which $R^4$ is hydrogen, $C_1$-$C_3$ alkyl, cyclopropyl-($C_1$-$C_3$ alkyl), $C_1$-$C_3$ alkyl substituted by 1 to 3 halogen atoms, and di-($C_1$-$C_3$ alkyl)-amino-($C_1$-$C_3$ alkyl); $R^5$ is hydrogen, hydroxy or carboxy; $R^6$ is a hydrogen or halogen atom; and $R^7$ is a hydrogen or halogen atom.

Further preferred are those in which $R^4$ is hydrogen, methyl, cyclopropylmethyl, 2,2,2-trifluoroethyl, and diethylaminoethyl; $R^5$ is hydrogen, hydroxy or carboxy; $R^6$ is a hydrogen or chlorine atom; and $R^7$ is a chlorine atom.

The explanations given above for "alkyl" and "halogen" apply here as well. In addition, the term "haloalkyl" is intended to include alkyl groups with multiple halogen substitutions as well as single substitutions, the multiple halogens being the same or different and preferably up to three per alkyl group. The term "carboxy" refers to the --COOH group. The term "salt" is intended to encompass both acid and alkali salts, examples of the latter being alkali metal salts, with sodium and potassium preferred.

Examples of benzodiazepines within the scope of formula II and useful in the present invention are as follows (generic name followed by Chemical Abstracts name and substituents for formula II):

diazepam; 7-chloro-1,3-dihydro-1-methyl-5-phenyl-2H-1,4-benzodiazepin-2-one; $R^4 = CH_3$, $R^5 = H$, $R^6 = H$, $R^7 = 7\text{-}Cl$ (the 7-position being defined as the position meta with respect to the carbon to which the -C=N- group is attached)
oxazepam; 7-chloro-1,3-dihydro-3-hydroxy-5-phenyl-2H-1,4-benzodiazepin-2-one; $R^4 = H$, $R^5 = OH$, $R^6 = H$, $R^7 = 7\text{-}Cl$
clorazepate dipotassium; 7-chloro-2,3-dihydro-2,2-dihydroxy-5-phenyl-1H-1,4-benzodiazepine-3-carboxylic acid, dipotassium salt; $R^4 = H$, $R^5 = COOH$, $R^6 = 2'\text{-}Cl$ (the 2′-position being defined as the ortho position on the lower phenyl ring), $R^7 = 7\text{-}Cl$
prazepam; 7-chloro-1-(cyclopropylmethyl)-1,3-dihydro-5-phenyl-2H-1,4-benzodiazepin-2-one;

$R^4 =$ 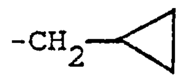 ,

$R^5 = H$, $R^6 = H$, $R^7 = 7\text{-}Cl$
lorazepam; 7-chloro-5-(o-chlorophenyl)-1,3-dihydro-3-hydroxy-2H-1,4-benzodiazepin-2-one; $R^4 = H$, $R^5 = OH$, $R^6 = 2'\text{-}Cl$, $R^7 = 7\text{-}Cl$
halazepam; 7-chlcro-1-(2,2,2-trifluoroethyl)-1,3-dihydro-5-phenyl-2H-1,4-benzodiazepin-2-one; $R^4 = -CH_2CF_3$, $R^5 = H$, $R^6 = H$, $R^7 = 7\text{-}Cl$
flurazepam hydrochloride; 7-chloro-1-[2-(diethylamino)ethyl]-5-(o-fluorophenyl)-1,3-dihydro-2H-1,4-benzodiazepin-2-one hydrochloride; $R^4 = -CH_2CH_2N(C_2H_5)_2$, $R^5 = H$, $R^6 = 2'\text{-}F$, $R^7 = 7\text{-}Cl$
Each of the examples in this list is a known, commercially available material.

The term "therapeutically effective amount" is used herein to denote any amount which will cause a substantial improvement in the condition for which the subject is being treated when administered to the subject either in a single dose or repeated doses over a period of time. The amount will vary with whether colic, teething or both are being treated, the degree of severity of the condition, the age and size of the subject, and the type and concentration of formulation applied. Appropriate amounts in any given instance will be readily apparent to those skilled in the art or capable of determination by routine experimentation.

The ratios of the two components of the present invention are not critical and may be varied over a wide range. Best results will generally be achieved when the mole ratio of antihistamine to benzodiazepine is about 0.05 to about 2.0, preferably about 0.1 to about 1.0, and most preferably about 0.1 to about 0.5.

Oral administration is preferred. A unit dose constituting a therapeutically effective amount for oral administration will generally fall within about 0.1mg to about 20mg of the combination. Formulations known to those skilled in the art for oral administration may be used. Exemplary formulations are solutions, suspensions and emulsions involving palatable liquids bearing a concentration whereby the unit dose amounts to approximately 0.1 to 10 milliliters.

As an optional variation, ethanol may be included in the formulation for enhanced activity.

The following example is offered for illustrative purposes, and is intended neither to limit nor define the invention in any manner.

## EXAMPLE

Two groups of patients, each group consisting of four infants or toddlers, each suffering from colic, teething or both, were treated as follows.

A formulation was prepared by grinding one 20mg tablet of diazepam (Roche Laboratories, Nutley, New Jersey) into a fine powder, and suspending the powder in 50mL of hydroxyzine oral suspension (Pfizer Laboratories, New York, New York). Each 0.5mL dose thus consisted of 0.2mg of diazepam and 2.5mg of hydroxyzine.

A 0.5mL dose of the formulation was administered to each patient at the onset of symptoms of colic or teething and repeated every 3 to 4 hours if the symptoms recurred. In all patients significant or complete relief of symptoms occurred within fifteen to thirty minutes.

The foregoing description is offered primarily for purposes of illustration. It will be readily apparent to those skilled in the art that numerous variations in both the formulations and their method of use beyond those described above may be made without departing from the spirit and scope of the invention.

## Claims

1. A composition for the treatment of a subject comprising an ataractic and a benzodiazepine having sedative hypnotic activity.

2. A composition in accordance with claim 1 in which said ataractic is a compound of the formula

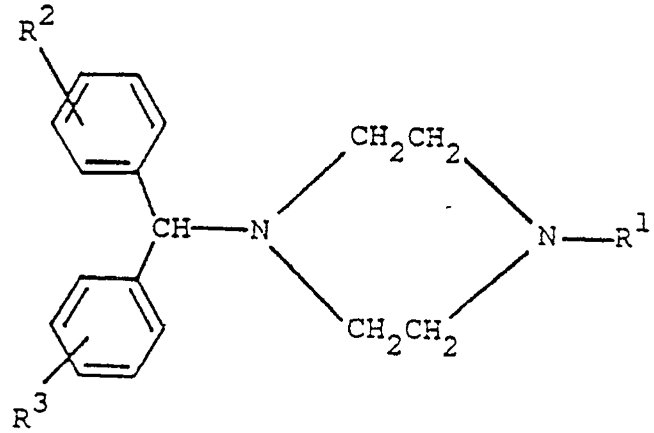

(I)

wherein $R^1$ is a member selected from the group consisting of $C_1$-$C_6$ alkyl, hydroxy-($C_1$-$C_6$ alkyl), hydroxy-($C_1$-$C_6$ alkoxy)-($C_1$-$C_6$ alkyl), phenyl-($C_1$-$C_6$ alkyl), and ($C_1$-$C_6$ alkyl)-phenyl-($C_1$-$C_6$ alkyl); and $R^2$ and $R^3$ are members independently selected from the group consisting of a hydrogen atom, a halogen atom, and $C_1$-$C_6$ alkyl; or an acid salt thereof.

3. A composition in accordance with claim 2 in which $R^1$ is a member selected from the group consisting of $C_1$-$C_3$ alkyl, hydroxy-($C_1$-$C_3$ alkyl), hydroxy-($C_1$-$C_3$ alkoxy)-($C_1$-$C_3$ alkyl), phenyl-($C_1$-$C_3$ alkyl), and ($C_1$-$C_4$ alkyl)-phenyl-($C_1$-$C_3$ alkyl); and $R^2$ and $R^3$ are members independently selected from the group consisting of a hydrogen atom, a chlorine atom, a bromine atom, and $C_1$-$C_3$ alkyl.

4. A composition in accordance with claim 2 in which $R^1$ is a member selected from the group consisting of methyl, hydroxyethoxyethyl and ($C_1$-$C_4$ alkyl)-phenylmethyl; $R^2$ is a member selected from the group consisting of a hydrogen atom, a chlorine atom and a bromine atom; $R^3$ is a hydrogen atom; and said acid salt is a member selected from the group consisting of a hydrochloride salt, a lactate salt and a pamoate salt.

5. A composition in accordance with claim 2 in which $R^1$ is hydroxyethoxyethyl, $R^2$ is 4-chloro, $R^3$ is a hydrogen atom, and said compound is the pamoate salt thereof.

6. A composition in accordance with claim 1 in which said benzodiazepine is a compound of the formula

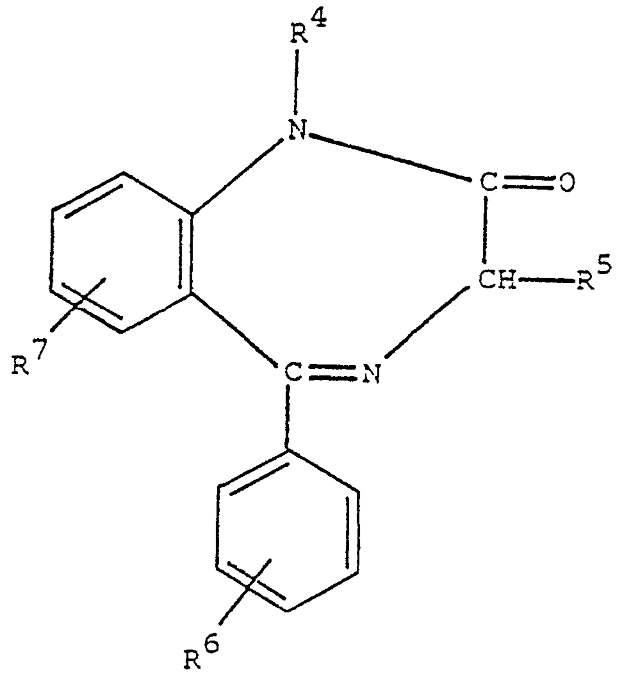

(II)

wherein $R^4$ is a member selected from the group consisting of hydrogen, $C_1$-$C_6$ alkyl, $C_3$-$C_6$ cycloalkyl, $C_1$-$C_6$ haloalkyl, amino-($C_1$-$C_6$ alkyl), ($C_1$-$C_3$ alkylamino)- (C,-$C_3$ alkyl), and di-($C_1$-$C_3$ alkyl)-amino-($C_1$-$C_6$ alkyl); $R^5$ is a member selected from the group consisting of a hydrogen atom, hydroxy, and carboxy; $R^6$ is a member selected from the group consisting of a hydrogen atom, a halogen atom, and $C_1$-$C_6$ alkyl; and $R^7$ is a member selected from the group consisting of a hydrogen atom, a halogen atom, and $C_1$-$C_6$ alkyl; or a salt thereof.

7. A composition in accordance with claim 6 in which $R^4$ is a member selected from the group consisting of a hydrogen atom, $C_1$-$C_3$ alkyl, cyclopropyl-($C_1$-$C_3$ alkyl), $C_1$-$C_3$ alkyl substituted by 1 to 3 halogen atoms, and di-($C_1$-$C_3$ alkyl) amino-($C_1$-$C_3$ alkyl); $R^5$ is a member selected from the group consisting of a hydrogen atom, hydroxy, and carboxy; $R^6$ is a member selected from the group consisting of a hydrogen atom and a halogen atom; and $R^7$ is a member selected from the group consisting of a hydrogen atom and a halogen atom.

8. A composition in accordance with claim 6 in which $R^4$ is a member selected from the group consisting of a hydrogen atom, methyl, cyclopropylmethyl, 2,2,2-trifluoroethyl, and diethylamino-ethyl; $R^5$ is a member selected from the group consisting of a hydrogen atom, hydroxy, and carboxy; $R^6$ is a member selected from the group consisting of a hydrogen atom and a chlorine atom, and $R^7$ is a chlorine atom.

9. A composition in accordance with claim 6 in which $R^4$ is methyl, $R^6$ is a hydrogen atom, $R^6$ is a hydrogen atom, and $R^7$ is a chlorine atom.

10. A composition in accordance with claim 1 in which said ataractic is hydroxyzine pamoate and said benzodiazepine is diazepam.

11. A composition in accordance with claim 1 in which the mole ratio of said ataractic to said benzodiazepine is about 0.05 to about 2.0.

12. A composition in accordance with claim 1 in which the mole ratio of said ataractic to said benzodiazepine is about 0.1 to about 1.0.

13. A composition in accordance with claim 1 in which said ataractic is hydroxyzine pamoate and said benzodiazepine is diazepam, and the mole ratio of said hydroxyzine pamoate to said diazepam is about 0.1 to about 1.0.

14. A composition in accordance with claim 1 in which said ataractic is hydroxyzine pamoate and said benzodiazepine is diazepam, and the mole ratio of said hydroxyzine pamoate to said diazepam is about 0.1 to about 0.5.

15. A composition for the treatment of teething and colic in accordance with any of claims 1, 2, 3, 4, 5, 6, 7, 8, 9, 10, 11, 12, 13 or 14 comprising an ataractic and a benzodiazepine having sedative hypnotic activity.

16. Use of a composition in accordance with any of claims 1, 2, 3, 4, 5, 6, 7, 8, 9, 10, 11, 12, 13, 14, or 15 for the manufacture of a medicament for the treatment of a subject suffering from a condition selected from

the group consisting of teething an colic.

European Patent Office

# EUROPEAN SEARCH REPORT

Application Number

EP 89 12 1515

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
| --- | --- | --- | --- |
| X | EP-A-0 150 721 (FIRMA PALIZ, ZIEGLER) * Claims 1-3 * --- | 1,6-9, 11,15 | A 61 K 31/55 // (A 61 K 31/55 A 61 K 31:495) |
| X | CHEMICAL ABSTRACTS, vol. 86, no. 15, 11th April 1977, abstract no. 100668t, Columbus, Ohio, US; J.M. CLIFFORD: "Determination of two benzodiazepine anticonvulsants in the plasma of rhesus monkeys when given intravenously, singly or together or in combination with a benzhydrylpiperazine anticonvulsant", & METHODOL. DEV. BIOCHEM. 1976, 5, 203-9 * Abstract * --- | 1-9 | |
| A | US-A-2 899 436 (UNION CHIMIQUE BELGE S.A., BELGIUM) * Column 1, lines 15-39 * --- | | |
| A | POLISH SOCIETY OF ELECTROENCEPHALOGRAPHY AND CLINICAL NEUROPHYSIOLOGY, vol. 45, no. 3, 1978, pages 7P-8P, NL; E. CZARNECKA et al.: "Clinical and experimental electroencephalographic investigations of the combined effect of ethyl alcohol with caffeine, hydroxyzine and diazepam." * Abstract * ----- | | TECHNICAL FIELDS SEARCHED (Int. Cl.5) |
| | | | A 61 K |

The present search report has been drawn up for all claims

| Place of search | Date of completion of the search | Examiner |
| --- | --- | --- |
| THE HAGUE | 18-07-1990 | BERTOCCHI C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)